(19) Europäisches Patentamt
European Patent Office
Office européen des brevets

(11) Publication number: **0 201 359**
**A2**

(12) # EUROPEAN PATENT APPLICATION

(21) Application number: **86400095.5**

(22) Date of filing: **17.01.86**

(51) Int. Cl.4: **A 61 K 31/47**, A 61 K 31/485

(30) Priority: **17.01.85 US 692309**

(43) Date of publication of application: **12.11.86 Bulletin 86/46**

(84) Designated Contracting States: **AT BE CH DE FR GB IT LI LU NL SE**

(71) Applicant: **MITSUBISHI CHEMICAL INDUSTRIES LIMITED, 5-2, Marunouchi 2-chome Chiyoda-ku, Tokyo 100 (JP)**

(72) Inventor: **Umezu, Kohei, 1-22-2 Kirigaoka Midori-ku, Yokohama-shi Kanagawa-ken (JP)**
Inventor: **Yuasa, Satoshi, 4-18-24 Soshigaya Setagaya-ku, Tokyo (JP)**
Inventor: **Morita, Yoshiharu, 1988-52, Kita-Hassaku-cho Midori-ku, Yokohama-shi Kanagawa-ken (JP)**
Inventor: **Taniguchi, Masao, 2-13-13 Ogawa, Machida-shi Tokyo (JP)**
Inventor: **Nomura, Tatsuo, 9809-61 Yatabedoai Hazaki-cho, Kashima-gun Ibaraki-ken (JP)**

(74) Representative: **Peaucelle, Chantal et al, S.C. Ernest Gutmann - Yves Plasseraud 67, boulevard Haussmann, F-75008 Paris (FR)**

(54) **Use of phthalide isoquinoline derivatives for treatments inhibiting the formation of lipid peroxides.**

(57) Disclosed are a method for the preparation of a drug for use in a treatment inhibiting the formation of lipid peroxide, the release of lysosome enzymes, the production of active oxygen, and/or the histamine release, a method for treatment of diseases associated with the formation of lipid peroxides, and, as an alternative of the invention, an improved method for the treatment of liver diseases, said methods comprising administering effective amounts of a compound of isoquinoline derivative represented by the general formula:

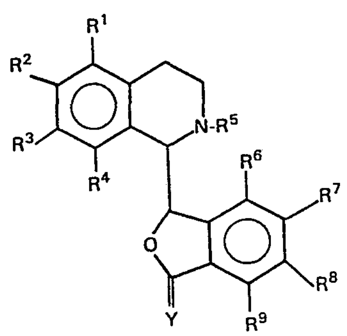

or a salt thereof, in which Y indicates an oxygen or sulphur atom; $R^1$, $R^2$, $R^3$, $R^4$, $R^6$, $R^7$, $R^8$ and $R^9$ independently indicate a hydrogen atom, a halogen atom, an alkyl group, a hydroxyl group, an alkoxy group, a halogen-substituted alkyl group, a thioalkoxy group, an amino group or a nitro group; or alternatively two adjacent groups $R^1$ to $R^4$ or $R^6$ to $R^9$ may together form a closed ring of alkylene group $>(CH_2)_m$ wherein m is 3 or 4, or alkylene dioxy group $\begin{matrix}-O\\-O\end{matrix}>(CH_2)_n$ wherein n is 1, 2 or 3; and $R^5$ indicates a hydrogen atom, an alkyl group, a hydroxyl group or an alkoxy group.

EP 0 201 359 A2

FIELD OF THE INVENTION

Disclosed are a method for the preparation of drug compositions for treatments inhibiting the formation of lipid peroxide, the release of lysosome enzymes, the production of active oxygen, and/or the histamine release, for a method for treatment of diseases associated with the formation of lipid peroxides, and, as an alternative of the invention, for an improved method for the treatment of liver diseases.

BACKGROUND OF THE INVENTION

(i) Inhibition of Formation of Lipid Peroxide.

It is well known that a cell membrane, which consists of a double layer of lipid, becomes more fragile when the lipid in it undergoes peroxidation and changes in the structure of the cell membrane occur. Pharmaceutical drugs that inhibit the formation of lipid peroxide are therefore considered to be effective in maintaining both normal conditions of the cell membrane and its stability to external stimuli.

For instance, lipid peroxide has recently been considered as a factor responsible for causing arteriosclerosis or arteriosclerotic-type diseases. This is because basic studies on lipid peroxide have progressed considerably such that an extremely small quantity of lipid peroxide in serum can be quantitatively determined, which has made it possible to investigate the formation and the quantity of lipid peroxide as a clinical or experimental evaluative index in diseases related to arteriosclerosis, or in experimentally caused arteriosclerosis. See The Saishin Igaku (in Japanese), Vol. 36, p. 659 (1981). The implication of lipid peroxide in the pathological outbreak and development of arteriosclerosis has been demonstrated by many evidences resulting from clinical statistics, case reports, analysis of blood platelet functions, experimental

biochemical and pathological observations, animal experiments and others.

Some pharmacological functions of lipid peroxide have now been clarified. Lipid peroxide promotes coagulation and adhesion of blood platelets, and a radical shift from lipid peroxide gives rise to abnormally low density lipoproteins (modified LDL) which are easily ingested by macrophages. As a result, the formation of foamy cells is accelerated and cholesterol derived from LDL deposits on the arterial wall. In addition, it is also known that the modification of proteins in endothelial cells by lipid peroxide makes the cells damaged, dead and degraded. See Domyaku Koka ; The Journal of Japan Atherosclerosis Society, Vol. 8, p. 295 (1982).

From these evidences, if inhibition of the formatin of lipid peroxide can be performed, it would be expected to prevent the sclerosis of, for instance, blood vessels. Furthermore, besides the destruction of the cell membrane in which lipid peroxides are formed and an acute disorder of the cell tissue, when lipid peroxide released from the cell amounts to a high concentration in serum, lipid peroxide may cause the disorder of various peripheral tissues, atrophy of blood vessels and the coagulation of blood platelets through the formation of prostaglandin in the platelets. For example, extensive coagulation of blood platelets induces thrombosis, destruction of the lysosome membrane leads to inflammation and the damage of erythrocyte membranes causes hemolysis. It is also possible to induce pulmonary emphysema by disorders of alveolar membranes.

Therefore, a compound which may strongly inhibit the formation of lipid peroxide is considered to be effective in preventing and/or treating the above

disease conditions.

(ii) Inhibition of Lysosome Enzyme Release and Production of Active Oxygen.

Recently, some doubts have arisen for the prostaglandin theory, which has been hitherto regarded as an important theory for understanding the causes of chronic inflammations and, instead, an active oxygen theory has now been proposed.

While polymorphonuclear leukocytes and macrophages have a phagocytotic activity and are therefore considered as cells which digest foreign substances and destroy bacteria when they invade the body, these actions of polymorphonuclear leukocytes and macrophages against bacteria and/or antigen-antibody complexes cause the production of active oxygen $O_2^-$. or .OH, resulting the direct damage on the tissues and the release of lysosome enzyme.

There are many reports which disclose the close relationship between inflammation and lysosome and $O_2^-$. released from leukocytes. For example, active oxygen at the inflammatory site has been confirmed in the experiments of an inflammatory model animal, and inhibition of an animal's carrageenin edema, adjuvant arthritis and reverse Arthus reaction of rat by SOD (superoxide dismutase) has also been observed. Further, it has also been reported that SOD is effecftive in the local administration for a patient suffering from rheumatism, which is a typical chronic inflammation. See The Saishin Igaku (in Japanese), Vol. 35, No. 7, pp. 1343-1349 (1980), Y. Shiokawa et al.

At present, chronic rheumatoid arthritis, nephritis, false gout and the like are also considered to be chronic inflammations relating to lysosome. All these diseases cause abnormal release of lysosome enzymes resulting in inflammatory symptoms accompanied

with pain. Prolonged release of lysosome enzymes from cells may cause chronic inflammation. At the same time, active oxygen including $O_2^-$., which is produced by polymorphonuclear leukocytes, may also cause damage to the tissues and, as a result, enlargement of inflammatory site and chronicity can arise.

Therefore, if the release of lysosome enzymes and the production of active oxygen are effectively inhibited, this may presumedly lead to inhibition of accentuation of inflammatory chronicity.

(iii) Inhibition of Histamine Release.

It is well known that mast cells are basophilic granular cells which exhibit positive metachromasia to basic dyes distributing widely in connective tissues and which release histamine and leukotriene, which are mediators of allergic reaction.

Connective tissues have hitherto been considered to have only a single function as a support of an organism. Now, connective tissues may be considered to be a means for controlling various actions of cells : for example, supply of nutrients to cells ; discharge of cellular metabolites ; preservation of the cell environment ; protection of cells from exogenous invasions by providing an inflammatory site ; or the like. On the other hand, mast cells *per se* have now re-recognized as an important cell bearing a role in various physiologic actions in the course of recent development of cell biology and biochemistry. See Taisha ; (Metabolism and Disease), Vol. 13, No. 5, a special number featuring articles on mast cells, Nakayama Shoten, Japan (in Japanese).

For example, new findings have recently been accumulated on pathogenesis of ulcerous colitis. The main symptom of this disease, that is, an appearance of persistent inflammation in the colic mucous membrane,

may be based on the breakdown of the protective mechanism against invasion of an antigen such as a bacterium through the enteron and on the establishment of autoimmunity to the colic mucous membrane.

This disease is associated with both immediate allergy and delayed allergy. In the acute phase, pathema is based on the establishment of the immediate allergy in the colic mucous membrane. Indeed, a high concentration of histamine in plasma, the increase of eosinophils in the colic mucous membrane, the decrease of mast cells and the like are observed in this phase. Correspondingly to the immediate allergy, induction of vasostimulant substances and disorder of microcirculation in the enteron may be caused, and various symptoms of the acute phase appear due to the increase of permeability in the colic mucous membrane and the reinforcement of myoparalysis.

It has already been reported cromoglycate, which may inhibit the degranulation of mast cells, i.e. which is an inhibitor of the histamine release, can be effective for ulcerous colitis. In other words, a compound which may exhibit a strong inhibitory activity to the histamine release from mast cells can be expected to be especially effective for the treatment of ulcerative colitis.

Recently, it has been known that histamine acts an important role not only as a mediator of acute inflammation in the primary stage but also as a regulator of chronicity of the inflammation. For instance, fibroblasts have a receptor (or receptors) to histamine and it may be considered that histamine accelerates the production of collagen. On the other hand, the fact that mast cells increase in the fibrous tissues has also been confirmed.

Accordingly, it may be considered that, if any

action is effected to inhibit the histamine release, acceleration of fibrosis i.e. increase of the collagen production may be indirectly inhibited in the tissues with a high degree of chronicity in inflammation. Compounds which would possess the last mentioned properties and, simultaneously, be capable of inhibiting the formation of lipid peroxide, the release of lysosome enzyme and the production of active oxygen would accordingly be useful at reasonable dosages in the treatment, for example, of diseases accompanied with abnormal propagation of fibers, particularly the effective inhibition of pulmonary fibrosis, the treatment of keroid after an operation and the effective inhibition of abnormal propagation of fibers due to an injury, or the like.

A first object of the invention was thus to provide a class of compounds which could have such an array of distinct properties. In their search for such compounds the present inventors happened to finally address themselves to a compound known as tritoqualine, a compound which can be designated by the following chemical name :

7-amino-4,5,6-triethoxy-3-(5,6,7,8-tetrahydro-4-methoxy-6-methyl-1,3-dioxolo(4,5-g)isoquinolin-5-yl)phthalide, $C_{26}H_{32}N_2O_8$, represented by the following structural formula (I) :

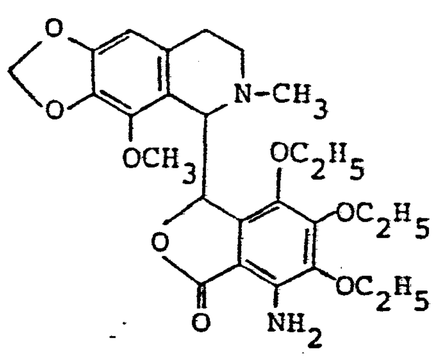

(I)

Tritoqualine is a compound which is used clinically as a drug effective against allergic diseases classified as type I-allergies such as pollinosis, asthma and urticaria. As a matter of fact tritoqualine is known as having inhibiting properties with respect to the activity of histidine decarboxylase, an enzyme known to catalyse the decarboxylation of histidine into histamine.

K. Ishii et al. also made a clinical study of the therapeutic effects of tritoqualine as a histidine decarboxylase inhibitor on chronic active hepatitis, when hyper-histaminemia was suspected to be a cause of liver injury in patients afflicted with chronic active hepatitis. Indeed they reported (Nihon Shokakibyo Gakkai Zasshi, 74 (9), 1187-1194 (1977) and in Gastroenterologia Japonica, 13 (2), 105-110 (1978)) results obtained upon administering daily dosages ranging from 600 to 1600 mg of tritoqualine to 13 patients with chronic active hepatitis. A marked improvement of liver functions and a reduction of fibrosis with cellular infiltration were observed on biopsied liver specimens, taken from the patients, particularly at dosage levels of 1200-1600 mg/day of tritoqualine.

Although K. Ishii et al. mentioned that no obvious side-effects were observed, the last mentioned result was however not confirmed when similar treatment protocols were extended to a larger number of patients. As a matter of fact side-effects, sometimes quite severe, more particularly dizziness and nausea, were observed in a substantial proportion of the treated patients, even though others appeared to have a better tolerance with respect to the relatively high dosage of the drug. These differences of tolerance were at first unexplained.

Thus among the several objects of this invention

a main one was to remedy at least in part to the difficulties which were encountered in the broader therapeutical program oriented in the direction of patients afflicted with hepatic diseases, more particularly to find ways to overcome the side-effects which had proven to develop in a substantial proportion of the tritoqualine-treated patients.

A more general object of the invention was the opening of new fields of use of tritoqualine and chemically homologue compounds, i.e. for the treatment of diseases linked to the excessive production *in vivo* of lipid peroxides, more particularly blood vessel sclerosis and related diseases, including diseases bringing also into play uncontrolled release of lysosome enzymes and overproduction of free oxygen and/or excessive histamine production.

Further objects of this invention are in relation to the use of tritoqualine and of chemical homologues thereof for the production of pharmaceutical compositions more specifically oriented towards the new fields of use which are considered within the frame of this disclosure, as well as of pharmaceutical compositions having an improved suitability for the treatment of some of the diseases contemplated in the past in relation to tritoqualine, but with at least reduced side effects or even no side effects at all.

SUMMARY OF THE INVENTION

It has now been found that all biological properties which have been mentioned hereabove are simultaneously present in the compounds of formula I. This will be illustrated by the examples disclosed hereafter.

First embodiments of the present invention provide a method for treatment the production of drug composition for treatments which comprises (i)

inhibiting the formation of lipid peroxide, (ii) inhibiting the lysosome enzyme release and the production of active oxygen, and/or (iii) inhibiting the histamine release, which treatments comprise administering a compound or isoquinoline derivative represented by the general formula :

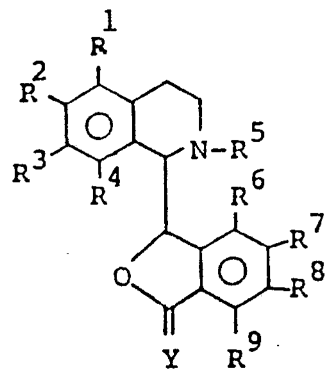

(I)

or a salt thereof, in which Y indicates an oxygen or sulphur atom ; $R^1$, $R^2$, $R^3$, $R^4$, $R^6$, $R^7$, $R^8$ and $R^9$ independently indicate a hydrogen atom, a halogen atom, an alkyl group, a hydroxyl group, an alkoxy group, a halogen-substituted alkyl group, a thioalkoxy group, an amino group or a nitro group ; alternatively two adjacent groups of $R^1$ to $R^4$ or $R^6$ to $R^9$ together may form a closed ring of alkylene group $>(CH_2)_m$ wherein m is an integer of 3 or 4, or alkylene group $\begin{matrix}-O\\-O\end{matrix}\!>(CH_2)_n$ wherein n is an integer of 1, 2 or 3 ; and $R^5$ indicates a hydrogen atom, an alkyl group, a hydroxyl group or an alkoxy group.

A second embodiment of this invention provides a method for the production of drug compositions for the treatment of liver diseases comprising administering a compound according to the above formula, particularly tritoqualine, orally and at least once a day, preferably several times a day, said administrations being adjusted and timed with respect to the meals in such a way that

total daily dosages of 100-700, preferably 300-700 mg will be effective. Other preferred daily dosages range from about 100 to about 600 mg, preferably from about 300 to 600 mg. Daily dosages ranging from about 150 to 300 mg have also been found to be effective when properly timed with respect to meals.

DESCRIPTION OF THE INVENTION

The invention will be hereinbelow described in more detail.

In the formula (I), the alkyl and halogen-substituted alkyl groups normally have 1 to 3 carbon atoms. Methyl and ethyl groups are particularly preferable. The alkoxy and thioalkoxy groups normally have 1 to 3 carbon atoms. The halogen atom is normally bromine or chlorine, and the halogen atom in the halogen-substituted alkyl group is normally fluorine, bromine or chlorine.

The isoquinoline derivative represented by the formula (I) may, for instance, be prepared by the following manner.

The compound (I) of which $R^6$ and/or $R^9$ is halogen atom or nitro group is obtainable by condensation reaction of a compound of the formula :

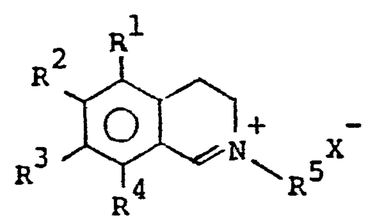

(II)

wherein $R^1$ to $R^5$ are defined as above and $X^-$ is an anion such as $I^-$, $Br^-$, $Cl^-$, $CH_3OSO_3^-$ or $OH^-$, and a compound of phthalide of the formula :

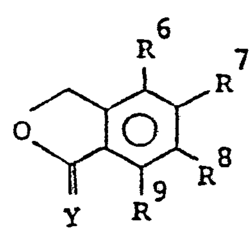

(III)

wherein $R^6$ to $R^9$ and Y are defined as above provided that at least one of $R^6$ and $R^9$ is halogen or nitro group. The compound (I) of which $R^6$ and/or $R^9$ is an amino group may be prepared by reducing of the nitro group of $R^6$ and/or $R^9$ of the above condensed product to the amino group.

The condensation reaction usually takes place by heating the reagents (II) and (III) in an alcoholic medium such as methanol and ethanol at a température of 40 to 100°C for 1 to 20 hours. The nitro group may be reduced to the amino group by means of any reducing agents for the nitro group, for instance, stannous chloride, tin-hydrochloride and iron-hydrochloride, or by means of catalytic hydrogenation.

The compounds of the formula (III), some of which are known, may be obtained by the nitration of halogenation of a phthalide compound of the formula :

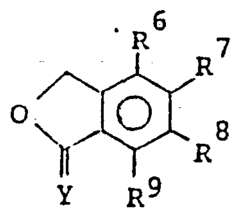

(IV)

wherein $R^6$ to $R^9$ and Y are defined as above provided that at least one of $R^6$ and $R^9$ is a hydrogen atom in a manner as disclosed in Justus Liebig's Annalen der Chemie, 98, 46 (1860).

The ammonium salts of substituted dihydroisoquinoline represented by the formula (II), some of which are also known, may be obtained by reacting a substituted dihydroisoquinoline of the formula :

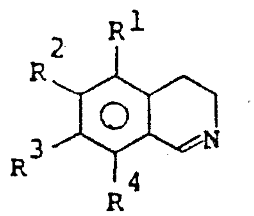

(V)

wherein $R^1$ to $R^4$ are defined as above with a compound of $R^5X$ in a usual manner. The compound of the formula (V) may be obtained according to the process as disclosed in Organic Reactions, 6, 74 (1951).

The compound of the formula (I), both $R^6$ and $R^9$ of which are other than halogen, nitro or amino group, may then be obtained by reducing a compound of the following formula :

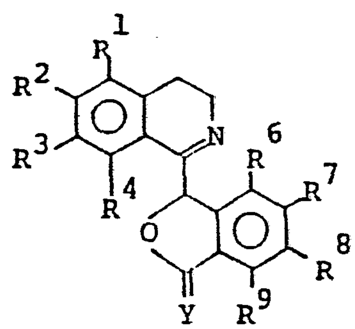

(VI)

wherein $R^1$ to $R^9$ and Y are defined as above. The reduced product is the compound (I) of which $R^5$ is the hydrogen atom. N-alkylation and N-hydroxylation of the thus reduced product may produce the compound (I) of which $R^5$ is the alkyl group and hydroxyl group, respectively. The compound (I) of which $R^5$ is alkoxy may be produced by N-hydroxylation of th reduced product followed by O-alkylation.

The reduction of compound (VI) is usually carried out by catalytic hydrogenation with palladium, platinum oxide or Raney nickel as catalyst, or by means of sodium borohydride in a medium of methanol, ethanol and etc. N-alkylation may be carried out by means of halogenated alkyl compound. In particular, N-methylation is carried out by the use of formalin-formic acid. N-hydroxylation can be carried out by means of oxidizing agents such as hydrogen peroxide. The O-alkylation of the N-hydroxyl derivative may be achieved, for instance, by the use of halogenated alkyl compounds.

The compound of the formula (VI) is obtainable by condensing a compound of the following formula :

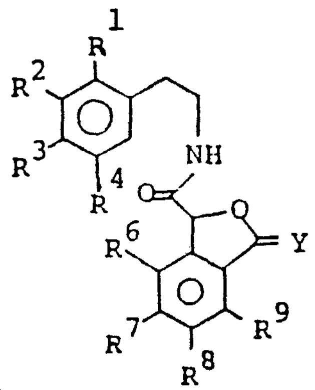

(VII)

wherein $R^1$ to $R^9$ and Y are defined as above according to the process as described in Journal fur praktische Chemie, 313, 923 (1971). The condensation reaction usually takes place by heating the compound (VII) with phosphorus oxychloride or phosphorus pentoxide as condensation agent either without a solvent or in a solvent such as benzene, toluene, methylene chloride or chloroform.

Alternatively, the compound of the formula (VII) may be obtained by the coupling reaction of a compound of the following formula :

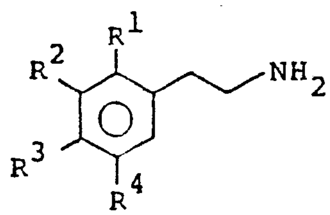

(VIII)

wherein $R^1$ to $R^4$ are defined as above with a carboxylic acid of the following formula :

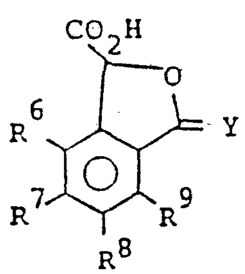

(IX)

wherein $R^6$ to $R^9$ and Y are defined as above. The coupling reaction may be carried out by means of the usual coupling agent, e.g., dicyclohexyl carbodiimide or alkyl chlorocarbonate, or by converting the carboxylic acid to acid chloride. The compound (IX) may be synthesized, for instance, by the process as described in Journal of Chemical Society, 127, 740 (1925).

Examples of the active compound (I) according to the present invention, which may be prepared in accordance with the foregoing description, are listed below :

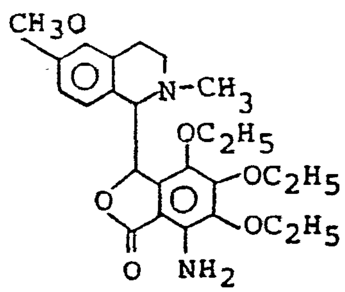

2-methyl-6-methoxy-1-(4,5,6-triethoxy-7-amino-3-phthalidyl)-1,2,3,4-tetrahydroisoquinoline

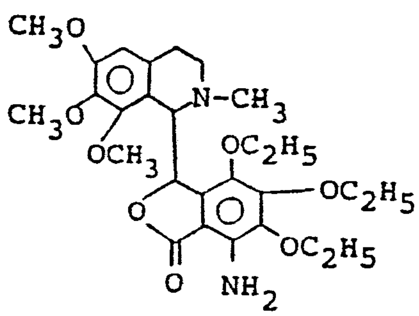

2-methyl-6,7,8-trimethoxy-1-(4,-5,6-triethoxy-7-amino-3-phthalidyl)-1,2,3,4-tetrahydroisoquinoline

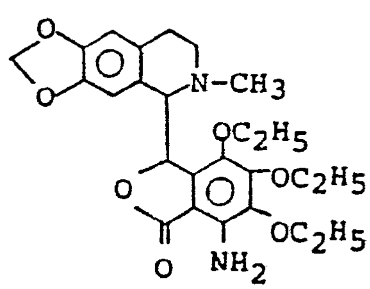

2-methyl-6,7-methylenedioxy-1-(4,5,6-triethoxy-7-amino-3-phthalidyl)-1,2,3,4-tetrahydroisoquinoline

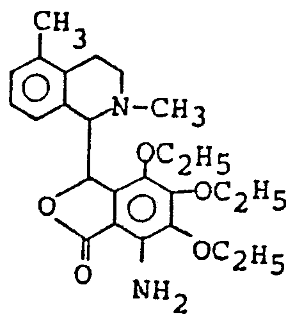

2,5-dimethyl-1-(4,5,6-triethoxy-7-amino-3-phthalidyl)-1,2,3,4-tetrahydroisoquinoline

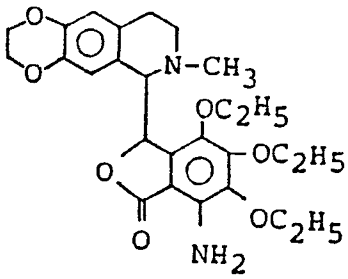

2-methyl-6,7-ethylenedioxy-1-(4,5,6-triethoxy-7-amino-3-phthalidyl)-1,2,3,4-tetrahydro-isoquinoline

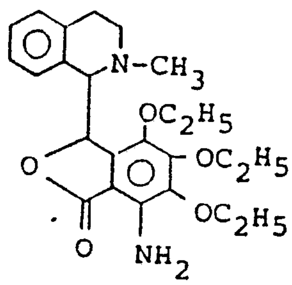

2-methyl-1-(4,5,6-triethoxy-7-amino-3-phthalidyl)-1,2,3,4-tetrahydroisoquinoline

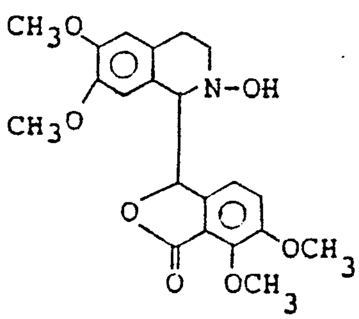

2-hydroxy-6,7-dimethoxy-1-(6,7-dimethoxy-3-phthalidyl)-1,2,3,4-tetrahydroisoquinoline

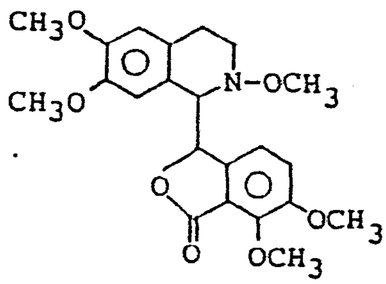

2,6,7-trimethoxy-1-(6,7-dimethoxy-3-phthalidyl)-1,2,3,4-tetrahydroisoquinoline

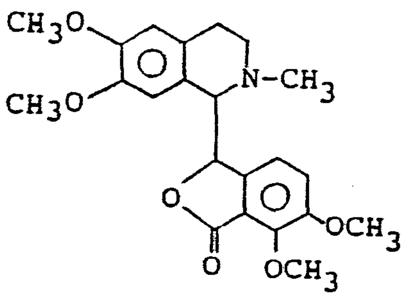

2-methyl-6,7-dimethoxy-1-(6,7-dimethoxy-3-phthalidyl)-1,2,3,4-tetrahydroisoquinoline

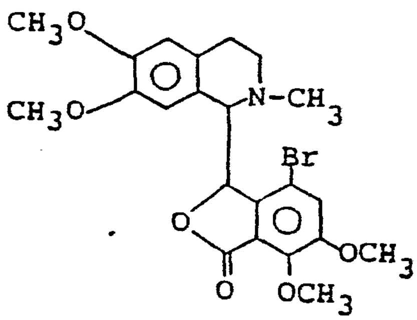

2-methyl-6,7-dimethoxy-1-(4-bromo-6,7-dimethoxy-3-phthalidyl)-1,2,3,4-tetrahydroisoquinoline

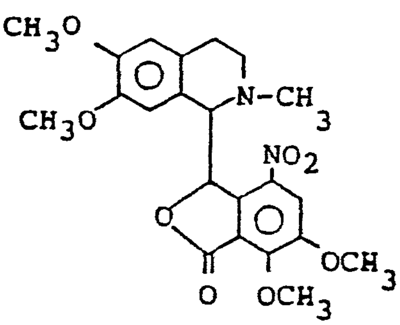

2-methyl-6,7-dimethoxy-1-(4-nitro-6,7-dimethoxy-3-phthalidyl)-1,2,3,4-tetrahydroisoquinoline

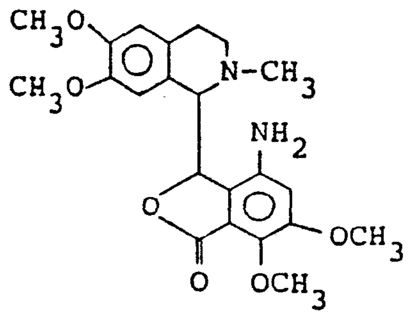

2-methyl-6,7-dimethoxy-1-(4-amino-6,7-dimethoxy-3-phthalidyl)-1,2,3,4-tetrahydroisoquinoline

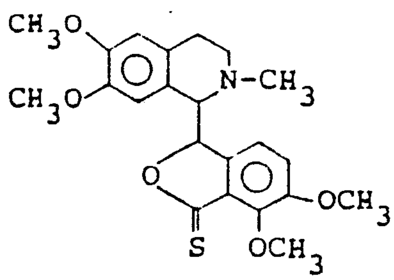

2-methyl-6,7-dimethoxy-1-(6,7-dimethoxy-3-(1-thiophthalidyl))-1,2,3,4-tetrahydroisoquinoline

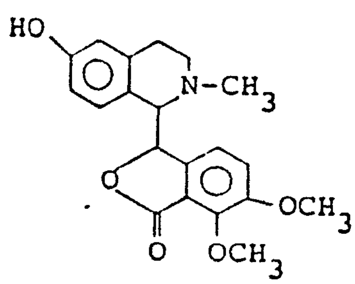

2-methyl-6-hydroxy-1-(6,7-dimethoxy-3-phthalidyl)-1,2,3,4-tetrahydroisoquinoline

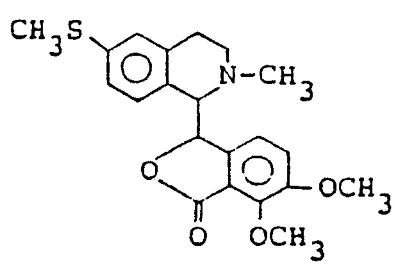

2-methyl-6-methylthio-1-(6,7-dimethoxy-3-phthalidyl)-1,2,3,4-tetrahydroisoquinoline

The present invention includes as the active compound a pharmaceutically acceptable salt of the above isoquinoline derivative, for example, inorganic salts such as those of hydrochloric acid, hydrobromic acid, hydroiodic acid, sulfuric acid, nitric acid or phosphoric acid ; or organic acid such as those of acetic acid, citric acid, maleic acid, fumaric acid, succinic acid, lactic acid, tartaric acid or benzoic acid.

Thus the invention concerns more particularly a method of production of a drug for therapeutical treatment in man or animal which comprises administering an amount of compound of formula I effective to inhibit the formation of lipid peroxide, which method is effective in maintaining the stability of cell membrane, and is useful in the treatment an prevention of diseases associated with lipid peroxide, for instance atherosclerosis, arterial sclerosis and arteriosclerotic diseases, abnormal aggregation of blood platelets, thromboses, pulmonar emphysema and disorders of peripheral tissue.

The invention further relates to a method of production of a drug for therapeutical treatment comprising administering an amount of compound of formula I which is effective to inhibit the release of lysosome enzymes and the production of active oxygen. Cell reactivity to the compounds of formula I is specific to inflammatory cells and, therefore, other cells such as blood cells, for example, erythrocytes or platelets are hardly affected. The active compounds have a strong affinity for inflammatory cells and inhibit the reaction of the inflammatory cells against external stimulations by entering into the cell membrane to be associated with lipid therein. In the inflammatory tissues, the compounds of formula I also inhibit chronicity of inflammation which is caused by

deterioration and/or prolongation of the inflammation due to tissue disorder by active oxygen, due to tissue destruction by lysosome enzyme, and the like. The compounds of formula I can be substituted with great advantage for SOD which, because of a high molecular weight, is impossible to be administered orally and there may be a risk of inducing an antigen-antibody reaction. Thus, the applicable range of SOD is limited. In the method of the present invention, on the contrary, the isoquinoline derivatives or salts thereof can be administered orally. Therefore, the method of the invention will be effective for the treatment of chronic rheumatoid arthritis, arthritis deformans, false gout, various chronic inflammations of the like.

The invention further relates the production of a drug for to a therapeutical method which comprises administering an amount of a compound of formula I which, in addition to having the above mentioned actions, is effective to strongly inhibit the histamine release by mast cells. Accordingly, the method of the invention is also for the production of drugs which will be effective against various diseases in which histamine is also involved. In other words, the isoquinoline derivatives of formule I or salts thereof are effective against diseases, for example ulcerative colitis, which may be now considered to be associated with mast cells according to the recent results of studies on the pharmacological mechanisms.

The method of the invention is further effective for the production of drugs for the inhibition of the pathological increase of collagen production in tissues affected with a high degree of inflammation chronicity, for the therapeutical treatment of diseases accompanied with abnormal propagation of fibers, particularly pulmonary fibrosis or consequent to injury or surgery

(treatment of keroids).

In the method for inhibiting the formation of lipid peroxide, particularly in excess of the amount that can be tolerated normally in a healthy host, the active isoquinoline derivative as described in the foregoings may be administered both orally and parenterally. Examples of the parenteral route are subcutaneous, intravenous, intramuscular and inter-peritoneal administration. The dose of the active compound depends on the age, state of health and body weight of the patient and the accompanied complication. However, the preferable dose may typically range from 50 to 2,000 mg, particularly from 100 to 500 mg per day, which may be administered, preferably orally, once or in several installments.

The oral administration may be effected in the form of tablets, capsules, powders or elixirs and, on the other hand, the parenteral administration in the form of sterilized liquids or suspensions. Non-toxic solid or liquid pharmaceutically acceptable carriers and adjuvants may be accompanied with the active ingredient.

As the solid carrier gelatin and the like are exemplified. The capsules, tablets or powders made with such solid carrier generally include 5 to 95 % by weight, preferably 25 to 90 % by weight of the active ingredient.

As the liquid carrier exemplified are water, vegetable oils such as peanut oil, soybean oil and sesame oil and any other pharmaceutically acceptable animal oils and synthetic oils. In general, physiological saline solution, dextrose or sucrose solution and glycols such as propylene glycol and polyethylene glycol are preferable as the liquid carrier. In particular, an injection solution containing the physiological saline solution includes 0.5 to 20 % by weight, preferably 1 to

10 % by weight of the active ingredient.

A liquid pharmaceutical for oral administration is a suspension or syrup including 0.5 to 10 % by weight of the active ingredient. The carrier therefor may be selected from flavourings, syrups or pharmaceutical acceptable micelle.

In the method according to second preferred embodiments of the present invention for the production of drugs for the treatment of liver diseases, the dosages are so adjusted that a compound of formula I, such as tritoqualine or a salt thereof, may orally be administered to a patient in a daily dose of 100-700 mg, or of 100-600, or of 100-500, or even 150-300 mg, and be effective at such dosages, subject to timing with respect to the meals. Preferred dosage ranges are from about 300 to about 700, or even from about 300 to about 600 mg per day.

The dose can be determined by a physician depending on the age, health condition and body weight of a patient to be treated, the kind and the number of the administration of comedication, if any, and the nature of desired effects. Preferably, the daily dose may be administered separately in 3 or 4 doses per day, most preferably after every diet.

If the compound of formula I, particularly tritoqualine, is administered immediately before diet or during diet, it will preferably be used in a liquid dosage unit form, i.e. a solution or suspension containing the compound with an average particle diameter of approximately 50 microns or less, preferably approximately 20 microns or less which has been obtained by subjecting the compound to micronisation and which is improved in absorbency, because absorbency or solubility of tritoqualine is less when administered before diet rather than after diet. Also, a solution of tritoqualine

in citric acid, malic acid or th like, which has an improved solubility, can preferably be utilized.

When tritoqualine or its salts is administered (most preferred form of administration) after diet, most preferably within one hour after the end of the meal, it has been found that particle size does not exert any influence and, therefore, any dosage form can be selected. Thus, tablets, capsule, powder, parvule, granule, elixir and the like containing compound of formula I in association with a solid or liquid, non-toxic, pharmaceutically acceptable carrier, can be used.

As important is the fact that, when the administration takes place in the above preferred conditions, an optimum activity is obtained within the low ranges of daily dosages which have been indicated.

By administering the specified amount of tritoqualine, side effects can be inhibited to a minimal degree and, therefore, administration after diet is possible while an improvement of liver disorders can be effected in an optimal degree.

It is considered that, according to the present inventors' studies, the thus described effects are also dependant on the following newly made observations :

The cell membrane consists of lipid double layers. If this lipid is peroxidized, the cell membrane structure changes and fragility of the cell membrane increases. However, tritoqualine or its salts inhibit the formation of lipid peroxide, whereby the double lipid layers of the cell membranes are maintained in or returned to the normal state, and protected against foreign stimulant. The compounds of formula I, preferably tritoqualine, may act a function for improving, particularly reducing the conditions of disorder in the liver cell membrane.

On the other hand, the functions of the

compounds of formula I of inhibiting activities on the cells in various inflammations and activating properties of liver functions which have been observed in vitro, are also expected to be effective in vivo. Accordingly, the advantageous effects on the treatment of liver diseases of tritoqualine and its homologues and the fact that they may be used under the above mentioned rather low dosage ranges, may be considered as resulting from a combination of the above-mentioned mechanisms. These results are all the more remarkable as the "dilution effect" of the diet absorbed by the patients could have led one to expect that the effective dosages would, after diet, be higher rather than lower than the dosages suggested by the relevant prior art.

Thus, the method according to the invention for the production of drugs for the treatment, in man or animal, provides drugs which will be effective to cause recovery or improvement of the liver functions by recovery and/or regenerating of the number and functions of the liver cells of and to provide for the protection against fragility of the parenchymal cell membranes in patients with acute or chronic hepatitis. The method of the invention is also effective for the production of drugs for the prevention or treatment of drug-induced poisoning conducive to liver diseases, particularly chronic diseases. The method of the invention is thus also effective for opposing recurrent or chronic liver diseases or for preventing liver diseases from becoming acute, recurrent or chronic.

The invention thus also relates to what can be termed a composition of matter combining meal food and a drug principle for combined absorption with said food by a patient afflicted with liver diseases, wherein the dosage of the drug is adjusted such as to provide substantially for the avoidance of the abovesaid

side-effects upon daily absorption by a patient afflicted with the abovesaid liver diseases of the amounts of said composition of matter (or of the components thereof) required for meeting his daily needs in food and wherein the absorption of said drug is timed such with respect of the absorption of the food of said composition of matter as to permit the daily absorbed doses of th drug principle to be effective against said liver diseases, it being of course understood that said drug principle consists of the isoquinoline derivative represented by the above-defined general formula, preferably tritoqualine.

More particularly the composition of matter has a dosage of drug principle sufficiently low to avoid the abovesaid side effects, while at the same time being effective against said liver diseases, when administered as a single dose or as several sub-doses a day, within approximately one hour from the end of one of the meals or of from each of the corresponding number of meals in said day respectively.

Even more particularly the invention relates to a composition of matter combining meal food and a drug principle for combined absorption by a patient afflicted with liver disease, wherein said drug principle is adjusted in amounts such as to provide for a daily absorption ranging from about 100 to about 700, particularly from about 100 to about 600, or from about 100 to 500 mg per day, or even from about 150 to about 300 mg per day of said drug principle by said patients. Advantageously said amounts are adjusted rom about 300 to about 700, or from about 300 to about 600 mg per day.

Compositions containing the compounds of formula I which can be used efficently for the treatment of liver diseases are illustrated hereafter in a more detailed manner in relation to tritoqualine considered

by way of preferred example of active compounds.

An illustrative example of the solid carrier is a conventional gelatin capsule, which is preferably a light-shielding capsule containing titanium oxide when use is made of active compounds, which, like tritoqualine, are unstable to light. The effective component, namely tritoqualine, may be subjected to tabletting or microencapsulation with or without one or more adjuvants, as well as one or more suitable carriers. Considering unstability of tritoqualine, a material for an envelope of a powder, parvule or granule may preferably be a composite film laminated with aluminium.

The preferable solution may be a suspension or syrup containing 0.5 - 10 % by weight of the effective component. A carrier which may be used is a perfume, syrup, pharmaceutical micelle or the like.

The tablet or capsule of the present invention may preferably be a pharmaceutical composition comprising, in parts by weight :

| | |
|---|---|
| (a) tritoqualine | 100 |
| (b) an excipient | 10 - 200 |
| (c) a disintegrant | 10 - 100 |
| (d) a binder | 2 - 10, and |
| (e) a lubricant | 4 - 20. |

Examples of the excipient include a starch such as corn starch, potato starch and the like, a sugar such ans mannitol, sorbitol, lactose and the like, and an inorganic salt such as calcium hydrogenphosphate, calcium carbonate and the like. Preferable excipients which may be used in the invention are starches.

Illustrative examples of the disintegrant include a cellulose such as calcium carboxymethylcellulose, avicel and the like, and a starch such as hydroxypropyl starch and the like. Celluloses may be preferred.

An example of the binder which may be used in the invention includes a starch such as potato starch, corn starch and the like, a cellulose such as hydroxypropyl cellulose, hydroxypropyl methylcellulose and the like, polyvinyl alcohol, and polyvinylpyrrolidone. Celluloses are preferably used.

An illustrative example of the lubricant includes higher fatty acid salts such as magnesium stearate, calcium stearate and the like, and silicates such as talc and the like, which are preferable in the present invention.

A preferable composition, particularly in the form of a tablet or capsule, of the invention comprises, in parts by weight :

| | |
|---|---|
| (a) tritoqualine | 100 |
| (b) a starch as an excipient | 10 - 200 |
| (c) a cellulose as a disintegrant | 10 - 100 |
| (d) a cellulose as a binder | 2 - 10, and |
| (e) as a lubricant, | |
| a higher fatty acid salt | 2 - 10, and |
| a silicate | 2 - 10. |

More preferable tablet or capsule comprises, in parts by weight :

| | |
|---|---|
| (a) tritoqualine | 100 |
| (b) corn starch | 2 - 20 |
| (c) potato starch | 10 - 50 |
| (d) avicel | 10 - 40 |
| (e) cellulose | 3 - 6, and |
| (f) calcium stearate and/or magnesium stearate and talc, where the weight ratio of calcium and/or magnesium stearate to talc is preferably 1 : 0.1 - 5 | 2 - 10. |

In the pharmaceutical composition of the present invention, the weight ratio of tritoqualine to the solid

carrier is preferably in the range of 1 : 0.2 - 6, more preferably in the range of 1 : 0.3 - 3.

In order to prepare a particulate material of the invention, a dry granulation in a fluidized bed may preferably be applied. In such a process, tritoqualine, the excipient and the disintegrant are first mixed. To the resultant mixture is added the binder which has previously been prepared in an aqueous solution with a concentration of 5 - 15 %, and then the materials are subjected to granulation. The thus obtained granule is mixed with the lubricant and the resulting mixture is pressed into a tablet or the mixture is filled into a capsule.

Since tritoqualine is unstable to the light as stated hereinabove, it is desirable to shield the tablet from the light by providing a coating thereon. An illustrative example of such a coating comprises, in parts by weight :

| | |
|---|---|
| (a) hydroxypropyl methyl cellulose | 100 |
| (b) titanium oxide | 10 - 40 |
| (c) a plasticizer such as polyethylene glycol and the like | 10 - 40, and |
| (d) a lubricant such as talc etc | 5 - 20. |

Further, a small amount of an antifoaming agent such as silicone and 10 - 40 parts by weight of hydroxypropyl cellulose with a low substitution degree for preventing slime can be effectively added to the aforementioned composition. Also, a dye can be added if desired.

The composition thus obtained is dissolved or suspended into water to a total concentration of 5 - 15 %.

The amount of the coating effective for light-shielding may be 3 mg or more per $cm^2$ of the surface area of a tablet, preferably 5 mg or more depending on

the shape, weight and the like of a tablet.

In the granulation process in a fluidized bed, a better result will be obtained if the disintegrant such as avicel is first added in an amount of from one third to two thirds of the total amount to be required and then the remainder is added after granulation. If the total amount of the disintegrant is added before granulation, the growth of granules is slow in the granulation process in a fluidized bed resulting in longer time for granulation. On the contrary, when the disintegrant is added separatedly in twice, the granulation time is shortened while uniform granules are obtained and the resulting granules can be better tabletted. In addition, the resulting tablet exhibits a short disintegrating time and almost no change of the properties even under rigorous conditions in a test.

The granules can be better tabletted. In addition, the resulting tablet exhibits a short disintegrating time and almost no change of the properties even under rigorous conditions in a test.

The granule of the present invention preferably comprises, in parts by weight :

| | |
|---|---|
| (a) tritoqualine | 100 |
| (b) an excipient | 100 - 2,000 |
| (c) a disintegrant | 10 - 300, and |
| (d) a binder | 5 - 100. |

The excipient, disintegrant and binder are as illustrated hereinabove for the tablet or capsule.

A preferable granule comprises, in parts in weight :

| | |
|---|---|
| (a) tritoqualine | 100 |
| (b) corn starch | 200 - 1,000 |
| (c) hydroxypropyl cellulose with a low substitution degree | 20 - 150, and |
| (d) hydroxypropyl methylcellulose | 15 - 50. |

The preferable concentration of tritoqualine in the granule of the invention may be in the range of 1 - 50 % by weight.

When a coating for light-shielding is provided on the granule, a preferable coating comprises, in parts by weight :

(a) hydroxypropyl methylcellulose 100, and
(b) titanium oxide 50 - 200.

This coating may also contain 10 - 40 parts by weight of polyethylene glycol as a plasticizer, 50 - 200 parts by weight of magnesium stearate and titanium oxide as a lubricant, and 1 - 3 parts by weight of a dye. The preferable amount of the coating sufficient for imparting an effective light-shielding property to a granule is, per total amount of the granule, not less than 10 % by weight, preferably not less than 15 % by weight.

In the preparation of the granule of the invention, the necessary components mentioned above are mixed and water is added to the resultant mixture, which is then kneaded. The thus obtained mixture is then subjected to a cylindrical granulator provided with a screen, dried, size-adjusted with an oscillator and further sieved.

A powder of the present invention may be prepared only by mixing tritoqualine and an excipient such as described above without granulation. A preferable powder comprises, in parts by weight :

(a) tritoqualine 100, and
(b) an excipient 200 - 2,000.

A parvule of the invention may be prepared in the same manner as the tablet and granules for the capsule, i.e. by granulation in a fluidized bed. A preferable parvule comprises, in parts by weight :

(a) tritoqualine 100
(b) an excipient 200 - 2,000

(c) a binder 15 - 50, and

(d) a lubricant 3 - 25.

The excipient, binder and lubricant are as described hereinabove.

A more preferable parvule of the present invention comprises, in parts by weight :

| | |
|---|---|
| (a) tritoqualine | 100 |
| (b) corn starch | 100 - 500 |
| (c) potato starch | 100 - 500 |
| (d) cellulose | 6 - 30, and |
| (e) calcium and/or magnesium stearate and talc | 4 - 15. |

The tablet, capsule, powder, granule and parvule of the present invention contain the effective component, tritoqualine, in an amount of e.g. 50, 100, 150, 200 or 250 mg.

DESCRIPTION OF PREFERRED EMBODIMENTS

In order to illustrate more fully the invention, the following examples are provided. These examples should not be considered as a limitation on the scope of the invention.

Example 1.

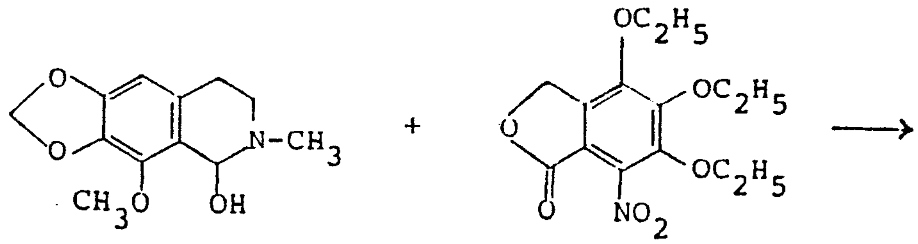

(1) (2)

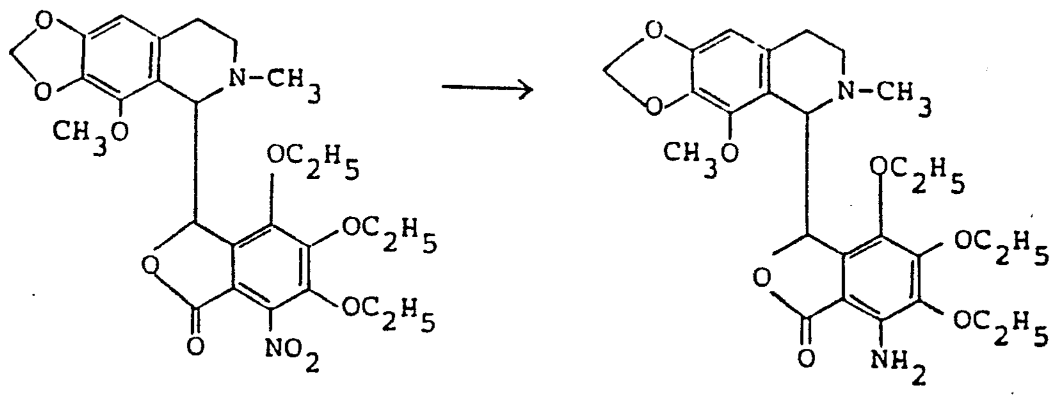

(3) (4)

To 26.1 g (0.11 mole) of cotarnine (1) were added 34.2 g (0.11 mole) of 4,5,6-triethoxy-7-nitro-phtalide (2) and 88 ml of methanol. The solution was heated under reflux for 10 hours. After cooling, 176 ml of methanol and 66 ml of methyl isobutyl ketone were added and stirred at room temperature for one hour. The undissolved crystal was filtered out, washed with 50 ml of methanol and dried. There was obtained 37.0 g of 2-methyl-6,7-methylenedioxy-8-methoxy-1-(4,5,6-triethoxy-7-nitro-3-phthalidyl)-1,2,3,4-tetrahydroisoquinoline (3) with a yield of 64 %.

To a mixture of 26.5 g (0.05 mole) of the product (3), 11.9 g (0.1 mole) of tin powder and 100 ml of acetone, 117 ml of 8 N hydrochloric acid was added over a period of 1.5 hours on a water bath for cooling. After stirring at room temperature for one hour, acetone

was evaporated under reduced pressure. The water layer was extracted twice with 150 ml of dichloroethane and to the combined dichloroethane layer was added gradually 150 ml of 10 % aqueous solution of sidum hydroxide. The dichloroethane layer was separated and washed twice with 100 ml of 1 % aqueous solution of ethylenediaminetetraacetic acid disodium salt, then 100 ml of water. After drying over anhydrous magnesium sulfate, the solvent was distilled off. Recrystallization of the residue from methyl ethyl ketone gave 23.0 g (yield 92 %) of tritoqualine (4) : melting point of 180-181°C, and IR (KBr, $cm^{-1}$) = 3,470, 3,350, 2,990, 2,950, 2,900, 2,810, 1,740, 1,490, 1,380, 1,265, 1,090 and 1,035.

Example 2.

2-ethyl-6,7-methylenedioxy-8-methoxy-1-(4,5,6-triethoxy-7-nitro or amino-3-phthalidyl)-1,2,3,4-tetrahydroisoquinoline.

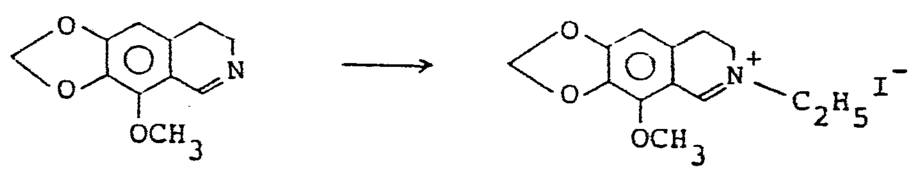

(1) (2)

In 15 ml of acetone, 4.04 g (19.7 mM) of 6,7-methylenedioxy-8-methoxy-3,4-dihydroisoquinoline (1) was dissolved and then 6.15 g (39.4 mM) of ethyl iodide was added thereto followed by heating under reflux for 1.5 hours. After cooling to a temperature of approx. 10°C, the precipitated crystals were separated by filtration, washed with 2 ml of acetone and dried to obtain 5.55 g of 2-ethyl-6,7-methylenedioxy-8-methoxy-3,4-dihydroisoquinolinium iodide (2) in a yield of 78 %. The compound (2) thus obtained has a melting point of 157°C after recrystallized from ethanol and has IR spectrum (KBr) at 2980, 1655, 1615, 1495, 1380, 1340, 1300, 1240, 1100 and

1045 $cm^{-1}$.

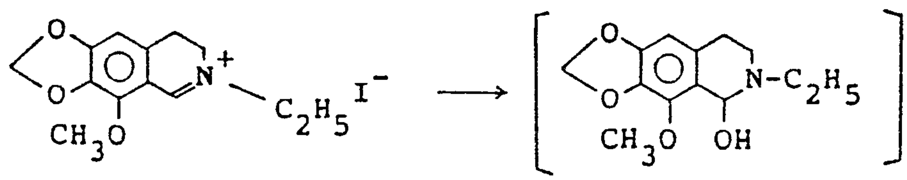

(2) (3)

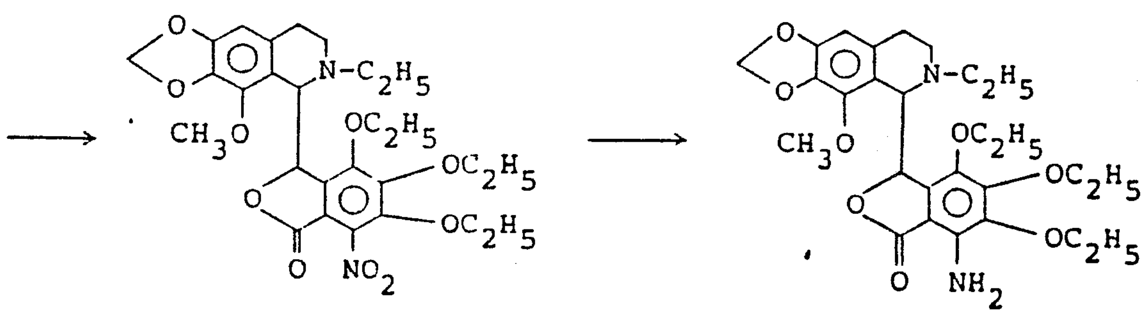

(4) (5)

To 4.24 g (11.7 mmol) of 2-ethyl-6,7-methylenedioxy-8-methoxy-3,4-dihydroisoquinolinium iodide (2) 60 ml of water was added, followed by adding 3 ml of 25 % aqueous solution of sodium hydroxide after cooling to approx. 15°C. The hydroxylized product (3) thus obtained was extracted twice with 40 ml and 20 ml of methylene chloride and, after drying over anhydrous magnesium sulfate, concentrated under reduced pressure.

To the residue was added 3.64 g (11.7 mmol) of 4,5,6-triethoxy-7-nitrophthalide and 8.5 ml of methanol, and then the mixture was heated under reflux for 3 hours. After cooling, 8.5 ml of methanol was further added, and then the mixture was stirred at room temperature for 1 hour. The precipitated crystals were separated by filtration, washed twice with methanol (2 x 4 ml) and dried to obtain 3.10 g of 2-ethyl-6,7-methylenedioxy-8-methoxy-1-(4,5,6-triethoxy-7-nitro-3-phthalidyl)-1,2,3,4-tetrahydroisoquinoline (4) in a yield of 49 %.

The product of compound (4) in an amount of 3.10 g (5.7 mmol) was dissolved in 14.8 ml of acetic acid, followed by adding 8.18 g of stannous chloride dihydrate in 9.0 ml of concentrated hydrochloric acid under water cooling. After stirring at room temperature for 3.5 hours, 110 ml of water and 70 ml of chloroform were added, followed by gradually adding 25 % aqueous solution of sodium hydroxide in an ice bath until the pH of the aqueous layer was approx. 11.

Celite-545 (8 g) was added and the reaction mixture stirred for 15 min, after which it was filtered through a layer of Celite-545. The Celite layer was washed with chloroform (2 x 50 ml), and the filtrate and washings were combined. The separated chloroform layer was then washed a saturated aqueous solution of sodium chloride (50 ml), dried over anhydrous magnesium sulfate, and the solvent was distilled off under reduced pressure.

To the residue was added 10 ml of methanol and 1.0 g of 85 % potassium hydroxide, and the mixture was heated under reflux for 2 hours. After cooling, the precipitated crystals were filtered off and washed with methanol (2 x 2 ml). They were then taken up in a mixture of chloroform (30 ml) and water (15 ml), and concentrated hydrochloric acid was added until the pH of the aqueous layer was approx. 2. After stirring for 10 min the pH of the aqueous layer was adjusted to approx. 11 with 25 % aqueous solution of sodium hydroxide, and it was separated. The aqueous layer was extracted with chloroform (10 ml), and the combined chloroform layers were washed with a saturated aqueous solution of sodium chloride (15 ml). After drying over anhydrous magnesium sulfate, the solvent was distilled off under reduced pressure, and the residue was recrystallized from ethanol to obtain 1.96 g of 2-ethyl-6,7-methylenedioxy-8-methoxy-1-(4,5,6-triethxy-7-amino-3-phthalidyl)-1,2,3,4-tetrahydroisoquinoline (5) in a yield of 67 %.

The physical properties of compound No. 5 are shown in Table I. Compounds Nos. 3, 4 and 6 in Table I were also prepared in a similar manner.

TABLE I

| Compound N° | Y | $R^1$ | $R^2$ | $R^3$ | $R^4$ | $R^5$ | $R^6$ | $R^7$ | $R^8$ | $R^9$ | Melting point (°C) | Ir (KBr) ($cm^{-1}$) |
|---|---|---|---|---|---|---|---|---|---|---|---|---|
| 1 | O | H | 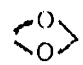 | | $CH_3O$ | $CH_3$ | $OC_2H_5$ | $OC_2H_5$ | $OC_2H_5$ | $NH_2$ | 180-181 | 3470, 3350, 2990, 2950, 2900, 2810, 1740, 1490, 1380, 1265, 1090, 1035. |
| 2 | O | H | 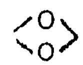 | | $CH_3O$ | $CH_3$ | H | H | $OCH_3$ | $OCH_3$ | 176 | ———— |
| 3 | O | H | $CH_3O$ | 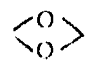 | | $CH_3$ | $OC_2H_5$ | $OC_2H_5$ | $OC_2H_5$ | $NH_2$ | 184-185 | 3525, 3420, 2980, 2940, 2900, 1730, 1445, 1380, 1050, 1030. |
| 4 | O | H | 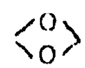 | | $CH_3O$ | $CH_3$ | $OC_2H_5$ | $OC_2H_5$ | $OC_2H_5$ | $NO_2$ | 147,5-149,5 | 2990, 1770, 1550, 1485, 1380, 1345, 1100, 1045, 1015. |
| 5 | O | H | 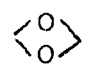 | | $CH_3O$ | $C_2H_5$ | $OC_2H_5$ | $OC_2H_5$ | $OC_2H_5$ | $NH_2$ | 158-159 | 3520, 3410, 2980, 2930, 2900, 1725, 1480, 1380, 1255, 1065, 1035. |
| 6 | O | H | $CH_3O$ | $CH_3O$ | H | $CH_3$ | $OC_2H_5$ | $OC_2H_5$ | $OC_2H_5$ | $NH_2$ | 142-143 | 3460, 3370, 2980, 2940, 1745, 1515, 1470, 1380, 1270, 1025. |

The biological, pharmacological properties of the compounds of this invention are further illustrated by the examples which follow. These examples which were carried out with compounds representative of the whole class should in no way be considered as having any limiting character. Some of the clinical uses of said compounds will be illustrated too in a non-limiting way.

Example 3.

Lipid peroxide was produced in vitro by using rat liver microsome. The microsome was prepared in a usual method from rat starved for 24 hours, and the final concentration thereof was adjusted to 15 - 18 mg proteins/ml.

To the thus prepared microsome was added $Fe(NO_3)_3$, ADP (adenosine diphosphate), NADPH (nicotinamide adenine dinucleotide phosphate), glucose 6-phosphate and glucose 6-phosphate dehydrogenase so that the final concentrations of these additives were 200 μM, 7 mM, 7.5 mM, 20 mM and 2.0 U.I., respectively.

The resultant mixture was incubated at 37°C to produce the lipid peroxide. The activity of compound No. 1 and vitamin E to inhibit the formation of the lipid peroxide was measured. The quantity of the lipid peroxide was determined in accordance with TBA method (thiobarbiturate method, "Biochemical Medicine", Vol. 15, p. 212 (1976)) and expressed in terms of MDA (malon dialdehyde) amount (nmol) in 1 mg of protein.

The results were shown in Table II.

TABLE II

| Compound (Dose : μM) | MDA Amount Produced (nmol/mg of protein) | |
|---|---|---|
| | Incubation Period | |
| | 5 min | 10 min |
| Control | 2.15 ± 0.14 | 2.76 ± 0.14 |
| Compound No.1 | | |
| 3.3 μM | 1.80 ± 0.12 | 2.25 ± 0.21 |
| 10 μM | 1.71 ± 0.12 | 1.80 ± 0.06 |
| 33 μM | 1.50 ± 0.05 | 1.62 ± 0.02 |
| 100 μM | 1.37 ± 0.02 | 1.37 ± 0.05 |
| Vitamin E | | |
| 10 μM | - | 2.37 ± 0.21 |
| 100 μM | 2.06 ± 0.001 | 2.62 ± 0.07 |
| 1000 μM | 2.06 ± 0.016 | 2.29 ± 0.14 |

Example 4.

Lipid peroxide was produced *in vitro* in cell membranes by using hepatocytes isolated by means of collagenase.

The isolated hepatocytes were prepared by the method of collagenase perfusion from the liver of rat which was fed for one week with phenobarbital in drinking water. The isolated hepatocytes ($1 \times 10^6$ cells/ml Hanks solution containing 1 % BSA and 10 mM HEPES, pH 7.4) were taken into a conical flask in which test compounds were placed. Then, the cells were incubated at 37°C and produced the lipid peroxide by the addition of carbon tetrachloride (5 mM).

The activity of the compounds shown in Table I and vitamin E to inhbit the formation of the lipid peroxide was measured. The amount of the lipid peroxide was determined in accordance with the method of Mihara et al. (Yakugaku Zasshi ; Journal of the Pharmaceutical Society of Japan, Vol. 102, No. 7, 670-677 (1982)) and expressed in terms of inhibition rate (%) of the formation of lipid peroxide after the addition of carbon tetrachloride.

The results were shown in Table III.

TABLE III

| Compound No. | Concentration (μM) | Free MDA (3 hours) % Inhibition |
|---|---|---|
| 1 | 10 | 77.3 |
| 2 | 10 | 42.4 |
| 3 | 10 | 76.9 |
| 4 | 10 | 28.6 |
| 5 | 10 | 68.6 |
| 6 | 10 | 47.6 |
| Vitamin E | 10 | 13 |

Example 5.

The lipid peroxide in the liver of the rat treated with $CCl_4$ increases. Carbon tetrachloride of 25 % V/V in olive oil was administered orally to rats by the dose of 2 ml/kg. After 24 hours, the amount of the lipid peroxide in the liver of the carbon tetrachloride-treated rat was increased to approx. 6 times higher than untreated rat. The amount of the lipid peroxide was determined by the method of Mihara et al. as in Example 3 and was expressed in terms of MDA amount (nmol) in 1 g of liver. The activity of compound No. 1 and vitamin E to inhibit the formation of the lipid peroxide was

measured. Compound No. 1 was orally administered to the carbon tetrachloride-treated rat in the form of suspension in the surfactant "Tween 80" by the amount of 25, 50 or 100 mg/kg/day nine days before the treatment of carbon tetrachloride.

The results were shown in Table IV.

TABLE IV

| Compound (dose : mg/kg) | MDA Amount Produced (nmol/g of liver) |
|---|---|
| Untreated | 59.6 ± 7.2 |
| Control fed with only $CCl_4$ | 328.2 ± 35.8 |
| Compound No.1 | |
| 25 mg/kg | 333.0 ± 54.3 |
| 50 mg/kg | 328.5 ± 50.8 |
| 100 mg/kg | 171.0 ± 23.6* |
| Vitamin E | |
| 50 mg/kg | 104.0 ± 18.4** |

* $P < 0.05$ ** $P < 0.01$

Example 6.

In 20 hours after injection of 10 ml of 1 % casein into the abdominal cavity of a guinea pig, many polymorphonuclear leukocytes appear in the cavity. An activity of the polymorphonuclear leukocytes can be determined in an *in vitro* system. Briefly stated, addition of phorbol myristate acetate, PMA, as a stimulant into a test tube containing a buffer and the polymorphonuclear leukocytes induces the production of $O_2^-$. by the polymorphonuclear leukocytes. At the same

time, the release of lysosome enzyme and the production of leukotriene are also induced. See Wei Hsueh et al., Nature, 290 (23), 710-713 (1981).

In such a system, the production of $O_2^-$. was strongly inhibited when PMA was added to the system after pre-incubation in the presence of the compounds shown in Table I.

Thus, measurements were carried out using rat polymorphonuclear leukocytes (PMN) induced by 1 % casein and PMA (phorbol-12-myristate-13-acetate) as a stimulant. First, 1,830 µl of phosphate buffer, pH 7,4, 50 µl of cytochrome C (Cyt C) in BSA, 20 µl of a sample (compound) and 50 µl of PMN were stirred at 37°C. After adding 50 µl of PMA to a final concentration of 125 ng/ml, the change of Cyt C reduction was traced by an absorption at 550 nm. The number of PMN per tube was $2 \times 10^6$ cells.

The results are shown in Table V.

TABLE V

| Compound No. | Conc. (μM) | % Inhibition (%) | IC50 (μM) |
| --- | --- | --- | --- |
| 1 | 3.3 | 15.4 | |
| | 10.0 | 53.9 | |
| | 33.0 | 58.8 | 15.9 |
| 3 | 3.3 | 34.9 | |
| | 10.0 | 59.9 | |
| | 33.0 | 66.2 | 7.2 |
| 5 | 3.3 | 49.2 | |
| | 10.0 | 65.8 | |
| | 33.0 | 60.3 | 1.8 |
| 6 | 3.3 | 0.9 | |
| | 10.0 | 14.0 | |
| | 33.0 | 41.1 | 45.1 |

Example 7.

Histamine release can be experimentally investigated *in vitro* with use of mast cells derived from rat's abdominal cavity. Thus, after the mast cells are pre-incubated in the presence of an IgE antibody, e.g. IgE antibody prepared by sensitization of rabbit with a protein derived from the epidermis of ascarid, addition of a corresponding IgE antigen, e.g. the protein prepared from the epidermis of ascarid, induces the release of histamine from the mast cells. Such release of histamine can also be induced by addition of an artificial stimulant compound 48/80, ATP (adenosine triphosphate) to mast cells.

A strong inhibiting activity to the histamine release of the compounds Nos. 1 and 3 shown in Table I was confirmed by the *in vitro* investigation using the

compound 48/80 as a stimulant and the protein extracted from the epidermis of ascarid.

Mast cells were collected from the abdominal cavity of male Wistar rats of 400 - 450 g in body weight in a conventional manner. After pre-incubation of 5 x $10^4$ cells in Krebs-Ringer buffer, pH 7.4, for 10 minutes, 0,1 μg/ml of the compound 48/80 was added. After incubation for an additional 10 minutes, the cells were removed by centrifugation at 3,000 rpm for 5 minutes. Thereafter, histamine in the supernatant was quantitatively measured according to Shore's method described in J. Phar. Exp. Ther., Vol. 127, 182 - 186 (1959). As a control, total amount of histamine contained in mast cell was measured. A ratio of the amount of histamine released after the treatment with the compound 48/80 as a stimulant to the total amount of histamine as the control was calculated according to the following equation :

$$\% \text{ Inhibition} = \frac{\text{amount of histamine after the treatment}}{\text{total amount of histamine in mast cell}} \times 100$$

The results are shown in Table VI.

TABLE VI

| Compound [*1] No. | Conc. (μM) | % inhibition | IC50 |
|---|---|---|---|
| | 3.3 | 4.7 | |
| 1 | 10.0 | 56.3 | 8.5 μM |
| | 33.0 | 90.7 | |
| | 3.3 | -1.5 | |
| 3 | 10.0 | 18.0 | 16.2 μM |
| | 33.0 | 91.3 | |

*1 : dissolved in 0.1N HCl.

The following examples 8-10 show the preparations of the tablets and granules of the present invention.

Example 8.

Fif teen kg of tritoqualine, 1.11 kg of corn starch, 4.65 kg of potato starch and 2.7 kg of avicel were mixed by a fluidized granulator-drier. The previously prepared 5 % aqueous solution of hydroxypropyl cellulose with a low substitution degree was sprayed onto the above obtained mixture to granulate, and then the resulting granules were dried. The dried granules were placed into a V-type mixer and 2.7 kg of avicel was added. Thereafter, 0.54 kg of magnesium stearate/talc with a weight ratio of 1:1 was further added and pressed into a tablet of 182 mg in weight.

On the surface of the obtained tablet, a coating comprising 6.1 parts by weight of hydroxypropyl methylcellulose, 1.2 parts by weight of polyethylene glycol 6,000, 1.8 parts by weight of titanium oxide, 0.9 parts by weight of talc, a small amount of silicone and 90 parts by weight of water was provided in an amount of

6 mg per $cm^2$.

The resulting tablet had a disintegrating time within 10 minutes. Further, there was no change under a fluorescent lamp of 1,200,000 lux.hr and under direct sun light.

Example 9.

Twenty kg of tritoqualine, 0.76 kg of corn starch, 2.4 kg of potato starch and 1.35 kg of avicel were mixed by a fluidized granulator-drier. To the mixture, 14 kg of a previously prepared 5 % aqueous solution of hydroxypropyl cellulose with a low substitution degree was sprayed to granulate followed by drying. After transferring the resultant granules to a V-type mixer, 1.35 kg of avicel was further added and mixed. Thereafter, 0.54 kg of 1:1 (by weight) magnesium stearate/talc was added and compressed into a tablet of 270 mg in weight.

A light-shielding tablet stable to the light was prepared by providing a coating in the same manner as Example 3.

Example 10.

After mixing 2,000 g of tritoqualine, 5,000 g of corn starch, 700 g of hydroxypropyl cellulose with a low substitution degree and 300 g of hydroxypropyl methylcellulose, 4,000 g of water was added and kneaded. After granulation in a cylindrical granulator provided with a screen of 0.8 mm in size, the granules were dried at 60°C for one hour and their sizes were adjusted by an oscillator. Sieving of the resulting granules gave granules with 16 - 60 mesh.

A coating was provided on the obtained granule in an amount of 18 % by weight. The coating comprised 5 parts by weight of hydroxypropyl methylcellulose, 7.5 parts by weight of titanium oxide, 1 part by weight of polyethylene glycol 6,000, 2 parts by weight of talc,

0.5 parts by weight of magnesium stearate and 74 parts by weight of ethanol. To the coated granule was added 2 % by weight of talc, and the resulting mixture was sieved to obtain a product of 16 - 60 mesh.

The resultant product had a disintegrating time of 1 - 2 minutes.

The following example shows an animal test using beagle dogs to which the drug was orally administered.

Example 11.

Tritoqualine was orally administered to male beagle dogs, 9 week old, body weight of 9.5 - 12 kg, Hazleton, after fast or 30 minutes after diet in a dose of 200 mg/kg.

Tritoqualine had been subjected to micronization by a jet mill PJM-100NP, NIHON NEWMATIC KOGYO KK, JAPAN (10 % or more of 3 microns or less, 80 % of 3-15 microns and 10 % or less of 15 microns or more ; average of 7 microns) or a screen of 100 mesh under. The obtained particles were suspended in aqueous 1 % Tween 80 solution before use. Thus, tritoqualine was used in the form of a slurry having a concentration of 100 mg/ml.

Before the administration, and 0.5, 1, 2, 3 and 5 hours after the administration, 1 ml of blood was taken out of the vein of front leg, placed into a BHA-coated centrifugal tube "VENOJECT" containing heparin, TERMO INC., and centrifuged. Hundred microliters of the thus obtained plasma was placed into a small glass test tube, which had been coated with 1 microgram of BHA. Into the tube were added 190 microliters of noscapine (100 micrograms in ml of acetonitrile) as an internal standard and 10 microliters of acetonitrile. After blending for one minute, the mixture was centrifuged at 3,000 rpm for 5 minutes. The resulting supernatant was subjected to HPLC under the conditions described below.

The quantitative measurements of tritoqualine in the plasma were carried out according to the internal standard method. The area under curve (AUC) was calculated by the trapezoidal rule.

Conditions of HPLC :

column ; ZORBAX C-8 4.6 x 250 mm

solvent ; 80 % aqueous acetonitrile, 2.5 mM, Pic B-7

flow rate ; 1.5 ml/min

detector ; fluorometer, HITACHI 650-10LC, Ex 340 nm, Em 440 nm

pump ; ALTEX A100.

The AUC of the concentrations in blood for 5 hours after the administration are shown in Table VII.

TABLE VII : AUC of tritoqualine concentration in blood for 5 hours.

| Dog No. | After fast | | 30 minutes after diet | | |
|---|---|---|---|---|---|
| | Micronisation 200 mg/kg | 100 mesh under 200 mg/kg | Micronisation 200 mg/kg | 100 mesh under 200 mg/kg | 100 mesh under 50 mg/ml |
| 1 | - | 125.3 | 674.5 | 861.5 | 186.8 |
| 2 | 76.8 | 281 | 720.5 | 702.3 | 176 |
| 3 | 500.5 | 13.5 | 522 | 1012.5 | 152.3 |
| 4 | 62 | 0 | 610 | 748.3 | 126.5 |
| 5 | 654.3 | 20.3 | 500.5 | 498.8 | 181.5 |
| | 323.4 ± 300 | 88.0 ± 118.9 | 605.5 ± 94.9 | 764.7 ± 190.8 | 164.6 ± 25.1 |

The following example 12 will illustrate the clinical investigations.

Example 12.

To patients suffering from chronic hepatitis tritoqualine was administered orally for 8 weeks continuously in a daily dose of 150 or 300 mg after every diet.

An improvement of liver functions was observed in 27 patients (44.3 %) among 62 patients administered in an amount of 150 mg, while 79 patients (61.2 %) among 135 patients administered in an amount of 300 mg.

Glutamate oxaloacetate transaminase (GOT) and glutamate pyruvate transaminase (GPT) were measured just before the first administration, and 4 and 8 weeks after the first administration.

The results are shown in Table VIII.

TABLE VIII

| Item | Dose (mg) | Number cases | Just before administration | 4 weeks after administration | 8 weeks after administration |
|---|---|---|---|---|---|
| GOT | 150 | 52 | 94.5±77.1 | 88.0±82.2 | 84.0±66.1 |
| (KU) | 300 | 108 | 104.9±50.7 | 96.2±51.1 | 89.8±44.6 |
| GPT | 150 | 52 | 94.8±54.2 | 80.6±91.0 | 69.8±65.1 |
| (KU) | 300 | 108 | 114.4±53.9 | 74.5±42.3 | 69.1±41.1 |

SIDE EFFECTS

During the above investigations, there were few cases where slight side effects such as dizziness and nausea, which were suspected to have a relation with the administered drug, appeared : in one case among 65 cases administered in a dose of 150 mg, i.e. 1.5 % ; and 8 cases among 137 cases in a dose of 300 mg, i.e. 5.8 %.

COMPARISON

When tritoqualine was administered in a dose of 600 mg in the same manner as described above, side effects appeared in 24 cases among 164 cases, i.e. 14.6 %.

Hence the reduction of the daily doses administered orally is accompanied by a drastic reduction of the side effects likely to be observed. It should be added that they were but transitory in the few instances where they appeared in those patients who received the low dosages in accordance with this invention.

Finally the invention further relates to the compounds encompassed by the abovesaid formula (I) and which are new per se, particularly those which can be designated by the above general formula I with the proviso that the latter does not extend to compounds 1, 2, 5 and 6 examplified hereinbefore.

In a same manner the invention further relates to the pharmaceutical compositions containing any of the compounds of formula I as limited by the above proviso clause, in association with a pharmaceutical vehicle, preferably with a vehicle suitable for the oral administration of said pharmaceutical composition. Accordingly by preferred new pharmaceutical compositions of the invention may be in the form of any of the preferred compositions which have been disclosed hereinbefore more particularly in relation to tritoqualine, except for the fact than any of the new compounds considered is substituted for tritoqualine. Particular preferred new pharmaceutical compositions of this invention contain also the preferred dosage ranges which have been defined hereinbefore, depending on the field of therapeutical use they are sought for.

The invention further relates to the packaged drugs including any of the above-defined compounds for

the abovesaid therapeutical methods, said packaged drugs comprising packaging means and, optionally, a separate notice, wherein either said packaging means or said notices or both comprise directions for the use of said drugs. The invention relates even more particularly to the packaged drugs in which said direction provide guidance for the user's sake, when said users are afflicted with the liver diseases referred to hereabove, said guidance aiming at enabling him to properly adjust the timing of absorption of the daily dosages referred to hereabove with respect to the meal absorption.

WE CLAIM

1. The use of the isoquinoline derivative defined hereafter or a salt thereof for the production of a pharmaceutical composition suitable for the treatment *in vivo* of diseases linked to the excessive production *in vivo* of lipid peroxides in an amount effective to inhibit the formation of lipid peroxide *in vivo*, wherein said isoquinoline derivative is represented by the general formula :

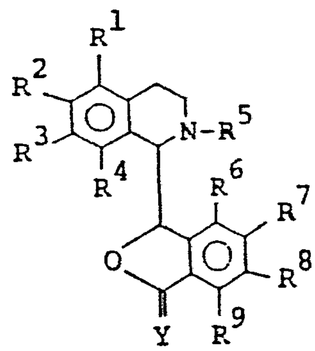

in which Y indicates an oxygen or sulphur atom ; $R^1$, $R^2$, $R^3$, $R^4$, $R^6$, $R^7$, $R^8$ and $R^9$ independently indicate a hydrogen atom, a halogen atom, an alkyl group, a hydroxyl group, an alkoxy group, a halogen-substituted alkyl group, a thioalkoxy group, an amino group or a nitro group ; or alternatively two adjacent groups of $R^1$ to $R^4$ or $R^6$ to $R^9$ may together form a closed ring of alkylene group $>(CH_2)_m$ wherein m is 3 or 4, or alkylene dioxy group 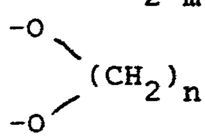 wherein n is 1, 2 or 3 ; and $R^5$ indicates a hydrogen atom, an alkyl group, a hydroxyl group or an alkoxy group.

2. The use of claim 1 wherein said isoquinoline derivative is, in said pharmaceutical composition, in an amount effective to inhibit the formation of lipid peroxide *in vivo*, when said pharmaceutical composition is administered to a host.

3. The use of claim 1 or 2 of the defined isoquinoline derivative for the production of a pharmaceutical composition suitable for the treatment of atherosclerosis, arteriosclerosis and arteriosclerotic-type diseases, abnormal aggregation and adhesion of blood platelets, the treatment and prevention of thrombosis, pulmonar emphysema and disorders of peripheral tissues.

4. The use of claim 1, wherein the amount of isoquinoline derivative is also effective to inhibit the production of active oxygen and the release of lysosome enzyme by polymorphonuclear leucocytes and macrophages.

5. The use of claim 1, wherein the amount of isoquinoline derivative is also effective to inhibit the histamine release by mast cells.

6. The use of claim 1 or 3, wherein said amount is adjusted as to provide for a daily dosage ranging from 50 to 2000 mg of said isoquinoline derivative, when intended for administration to a host.

7. The use of claim 1 or 3, wherein said dose ranges from 100 to 500 mg of said isoquinoline derivative per day.

8. The use of any of claims 1 to 7, wherein said isoquinoline derivative is tritoqualine.

9. The use of any of claims 1 to 7, wherein said isoquinoline derivative is selected from :

- 2-methyl-6,7-methylenedioxy-8-methoxy-1-(6-methoxy-7-methoxy-3-phthalidyl)-1,2,3,4-tetrahydroisoquinoline,
- 2-methyl-6-methoxy-7,8-methylenedioxy-1-(4,5,6-triethoxy-7-amino-3-phthalidyl)-1,2,3,4-tetrahydroisoquinoline,
- 2-methyl-6,7-methylenedioxy-8-methoxy-1-(4,5,6-triethoxy-7-nitro-3-phthalidyl)-1,2,3,4-tetrahydroisoquinoline,
- 2-ethyl-6,7-methylenedioxy-8-methoxy-1-(4,5,6-tri-

ethoxy-7-amino-3-phthalidyl)-1,2,3,4-tetrahydroisoquinoline,

- 2-methyl-6,7-dimethoxy-1-(4,5,6-triethoxy-7-amino-3-phthalidyl)-1,2,3,4-tetrahydroisoquinoline.

10. The use of the isoquinoline derivative defined hereafter or a salt thereof for the production of a pharmaceutical composition containing an amount of said isoquinoline derivative so adjusted as to be effective at a daily dosage ranging from 100 to 700 mg per day for the treatment of liver diseases when administered at the appropriate moment with respect to the meals, wherein said isoquinoline derivative is represented by the general formula :

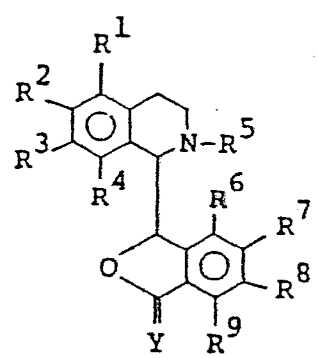

or a salt thereof, in which Y indicates an oxygen or sulphur atom ; $R^1$, $R^2$, $R^3$, $R^4$, $R^6$, $R^7$, $R^8$ and $R^9$ independently indicate a hydrogen atom, a halogen atom, an alkyl group, a hydroxyl group, an alkoxy group, a halogen-substituted alkyl group, a thioalkoxy group, an amino group or a nitro group ; alternatively two adjacent groups of $R^1$ to $R^4$ or $R^6$ to $R^9$ together may form a closed ring of alkylene group $>(CH_2)_m$ wherein m is an integer of 3 or 4, or alkylene dioxy group

$$\begin{matrix}-O\\ -O\end{matrix}\!\!>(CH_2)_n$$

wherein n in an integer of 1, 2 or 3 ; and $R^5$ indicates a hydrogen atom, an alkyl group, a hydroxyl group or an alkoxy group.

11. The use of claim 10 wherein the amount of said isoquinoline derivative in said pharmaceutical composition is so adjusted as to be effective at a dosage ranging from 100 to 500 mg per day, under the conditions defined in said claim 10.

12. The use of claim 10 wherein the amount of isoquinoline derivative in said pharmaceutical composition is adjusted to be effective at a daily dosage ranging from about 150 to about 300 mg under the conditions defined in said claim 10.

13. The use of claim 10 wherein the amount of isoquinoline derivative in said pharmaceutical composition is adjusted to be effective at a daily dosage ranging from about 300 to 700 mg under the conditions defined in said claim 10.

14. the use of claim 10 wherein the amount of isoquinoline derivative in said pharmaceutical composition is adjusted to be effective at a daily dosage from about 100 to about 600 mg, preferably from about 300 to 600 mg under the conditions defined in said claim 10.

15. The use of any of said claims 10 to 14, wherein the amount of said isoquinoline derivative is adjusted so as to be effective when said pharmaceutical composition is for administration within from about 0 to about 1 hour from the end of a meal or diet.

16. The use of any of said claims 10 to 14, wherein the total amount is divided in sub-amounts adjusted so as to be effective when said pharmaceutical composition is for administration within from about zero to about 1 hour from the end of every meal or diet.

17. The use of any of said claims 10 to 16, wherein said isoquinoline derivative is associated with a solid pharmaceutically acceptable carrier to form a solid composition containing said isoquinoline

derivative.

18. The use of any of claims 10 to 17, wherein the isoquinoline derivative is tritoqualine.

19. The use of any of claims 10 to 17, wherein said isoquinoline derivative is selected from the group of derivatives consisting of :

- 2-methyl-6,7-methylenedioxy-8-methoxy-1-(6-methoxy-7-methoxy-3-phthalidyl)-1,2,3,4-tetrahydroisoquinoline,
- 2-methyl-6-methoxy-7,8-methylenedioxy-1-(4,5,6-triethoxy-7-amino-3-phthalidyl)-1,2,3,4-tetrahydroisoquinoline,
- 2-methyl-6,7-methylenedioxy-8-methoxy-1-(4,5,6-triethoxy-7-nitro-3-phthalidyl)-1,2,3,4-tetrahydroisoquinoline,
- 2-ethyl-6,7-methylenedioxy-8-methoxy-1-(4,5,6-triethoxy-7-amino-3-phthalidyl)-1,2,3,4-tetrahydroisoquinoline,
- 2-methyl-6,7-dimethoxy-1-(4,5,6-triethoxy-7-amino-3-phthalidyl)-1,2,3,4-tetrahydroisoquinoline.

20. A composition of matter combining meal food and a drug principle for combined absorption by a patient afflicted with liver disease, wherein said drug principle is adjusted in amounts such as to provide for a daily absorption ranging rom about 100 to about 700 mg per day of said drug principle by said patient, wherein the absorption of said drug principle is timed with respect to the absorption of the food of the meals so as to make the drug principle effective in said patient to improve his health condition and wherein said drug principle consists of an isoquinoline derivative represented by the general formula :

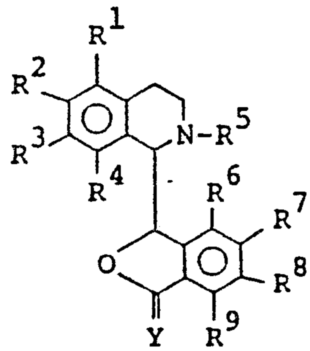

or a salt thereof, in which Y indicates an oxygen or sulphur atom ; $R^1$, $R^2$, $R^3$, $R^4$, $R^6$, $R^7$, $R^8$ and $R^9$ independently indicate a hydrogen atom, a halogen atom, an alkyl group, a hydroxyl group, an alkoxy group, a halogen-substituted alkyl group, a thioalkoxy group, an amino group or a nitro group ; alternatively two adjacent groups of $R^1$ to $R^4$ or $R^6$ to $R^9$ together may form a closed ring of alkylene group $>(CH_2)_m$ wherein m is an integer of 3 or 4, or alkylene dioxy group

$$\begin{matrix}-O \\ -O\end{matrix}\!\!>(CH_2)_n$$

wherein n in an integer of 1, 2 or 3 ; and $R^5$ indicates a hydrogen atom, an alkyl group, a hydroxyl group or an alkoxy group.

21. The composition of matter of claim 19 which is adjusted in amounts such as to provide for a daily absorption ranging from about 100 to about 500 mg per day.

22. The composition of matter of claim 19 which is adjusted in amounts such as to provide for absorption ranging from about 100 to about 600 mg per day, preferably from about 300 to about 600 mg per day.

23. The composition of matter of claim 19 wherein the adsorption of said drug principle is timed to take place from zero to about one hour from the end of the meal absorption.

24. The composition of matter of claim 19

wherein said drug principle is a solid composition containing said isoquinoline derivative in association with a solid pharmaceutically acceptable carrier.

25. The composition of matter of any of claims 19 - 24 wherein said isoquinoline derivative is tritoqualine.

26. The composition of matter of any of claims 19 - 24 wherein said isoquinoline derivative is selected from the group of isoquinoline derivatives consisting of :

- 2-methyl-6,7-methylenedioxy-8-methoxy-1-(6-methoxy-7-methoxy-3-phthalidyl)-1,2,3,4-tetrahydroisoquinoline,
- 2-methyl-6-methoxy-7,8-methylenedioxy-1-(4,5,6-triethoxy-7-amino-3-phthalidyl)-1,2,3,4-tetrahydroisoquinoline,
- 2-methyl-6,7-methylenedioxy-8-methoxy-1-(4,5,6-triethoxy-7-nitro-3-phthalidyl)-1,2,3,4-tetrahydroisoquinoline,
- 2-ethyl-6,7-methylenedioxy-8-methoxy-1-(4,5,6-triethoxy-7-amino-3-phthalidyl)-1,2,3,4-tetrahydroisoquinoline,
- 2-methyl-6,7-dimethoxy-1-(4,5,6-triethoxy-7-amino-3-phthalidyl)-1,2,3,4-tetrahydroisoquinoline.

27. A composition of matter combining meal food and a principle for combined absorption with said food by a patient afflicted with liver diseases, wherein the dosage of the drug is adjusted such as to provide substantially for the avoidance of the abovesaid side-effects upon daily absorption by a patient afflicted with the abovesaid liver diseases of the amounts of said composition of matter (or of the components thereof) required for meeting his daily needs in food and wherein the absorption of said drug is timed such with respect of the absorption of the food of said composition of matter as to permit the daily absorbed

doses of the drug principle to be effective against said liver diseases and wherein said drug principle consists of an isoquinoline derivative of the general formula :

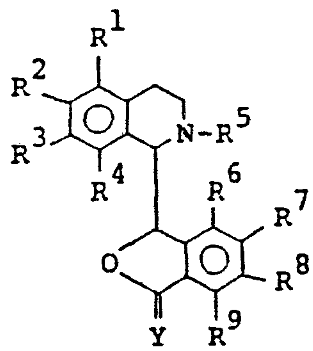

or a salt thereof, in which Y indicates an oxygen or sulphur atom ; $R^1$, $R^2$, $R^3$, $R^4$, $R^6$, $R^7$, $R^8$ and $R^9$ independently indicate a hydrogen atom, a halogen atom, an alkyl group, a hydroxyl group, an alkoxy group, a halogen-substituted alkyl group, a thioalkoxy group, an amino group or a nitro group ; alternatively two adjacent groups of $R^1$ to $R^4$ or $R^6$ to $R^9$ together may form a closed ring of alkylene group $>(CH_2)_m$ wherein m is an integer of 3 or 4, or alkylene dioxy group

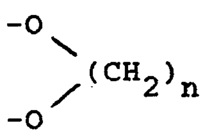

wherein n in an integer of 1, 2 or 3 ; and $R^5$ indicates a hydrogen atom, an alkyl group, a hydroxyl group or an alkoxy group.

28. The composition of matter of claim 27 which has a dosage of said drug principle sufficiently low to avoid the abovesaid side effects, while at the same time being effective against said liver diseases, when administered as a single dose or as several sub-doses a day, within approximately one hour from the end of one of the meals or from each of the corresponding number of meals in said day respectively.

29. The composition of claim 27 or 28, wherein said drug principle is tritoqualine.